# EUROPÄISCHE PATENTSCHRIFT

(11) **EP 0 774 207 B1**
(45) Veröffentlichungstag und Bekanntmachung des Hinweises auf die Patenterteilung: **14.02.2001**
(21) Anmeldenummer: 95118145.2
(22) Anmeldetag: 17.11.1995
(51) Int. Cl.: A01N 43/80, C07D 275/02

(54) **Verfahren zur Herstellung von bioziden oder biostatischen 3-Isothiazolinon-Zusammensetzungen**
Method for the preparation of biocidal or biostatic 3-isothiazolinone compositions
Procédé de préparation des compositions biocides ou biostatiques de 3-isothiazolinone

(43) Veröffentlichungstag der Anmeldung: 21.05.1997
(73) Patentinhaber: THOR GmbH, 67346 Speyer (DE)
(72) Erfinder: Baum, Rüdiger, D-68753 Waghäusel 1 (DE)
(74) Vertreter: DIEHL GLAESER HILTL & PARTNER

(56) Entgegenhaltungen:
- EP-A- 0 095 907
- EP-A- 0 606 986
- EP-A- 0 678 510
- US-A- 3 870 795
- US-A- 5 137 899
- DATABASE WPI Week 9122 Derwent Publications Ltd., London, GB; AN 91-159942 [22] XP002001139 & JP-A-03 095 103 (ICHIKAWA GOSEI) , 19.April 1991

## Beschreibung

Biozide sind unverzichtbare Bestandteile in vielen chemischen Stoffgemischen und Materialien des täglichen Gebrauchs, sowohl im gewerblichen als auch im nichtgewerblichen Bereich. Die Biozide verhindern, daß jene Stoffgemische und Materialien durch Einwirkung von Mikroorganismen verderben und dadurch unbrauchbar werden. Gleichzeitig schützen sie damit auch die Gesundheit der Verbraucher.

Biozide sind notwendig zur Konservierung von beispielsweise Reinigungsmitteln, Scheuermitteln, Waschmitteln, Poliermitteln, Anstrichmitteln, Klebstoffen, Kleistern, Papier, Pappen, Textilmaterialien, Leder, Bohrlochspülungen, Metallbearbeitungsflüssigkeiten, Latexemulsionen, Kunststoffen, Treibstoffen und Wasser in Kühlkreisläufen. Der bestimmungsgemäße Effekt der Biozide verleiht den damit versetzten Substraten eine wesentlich längere Lebensdauer, sowohl im geschlossenen Gebinde während der Lagerung und des Transports als auch während des Gebrauchs der Substrate.

3-Isothiazolinone sind bedeutende biozid wirkende Substanzen, welche die wichtigsten Eigenschaften eines modernen Biozids auf sich vereinigen. Besonders hervorzuheben ist das sehr breite Wirkungsspektrum gegenüber Bakterien, Schimmelpilzen, Hefen und Algen sowie die leichte Abbaubarkeit in der Umwelt. Je nach den Substituenten im Isothiazolinonmolekül unterscheiden sich die Isothiazolinone in ihrer Reaktivität und somit auch in ihrer Wirkung und/oder sind in ihrer Löslichkeit unterschiedlich.

Die Herstellung von 3-Isothiazolinonen ist bekannt und kann auf verschiedenen Wegen erfolgen. Beispielsweise beschreibt die US-A-3849430 zwei Alternativen. Gemäß der ersten Alternative wird ein Dithiodiamid durch Cyclisieren mit einem Halogenierungsmittel in das entsprechende 3-Isothiazolinon überführt. So entsteht beispielsweise aus 3,3'-Dithio-N,N'-dimethyldipropionamid durch Umsetzen mit Sulfurylchlorid ein Gemisch aus 5-Chlor-2-methylisothiazolinon und 2-Methyl-3-isothiazolinon. Gemäß der zweiten Alternative wird anstelle des Dithiodiamids ein Mercaptoamid verwendet. Man erhält dann beispielsweise durch Reaktion von N-Methyl-3-mercaptopropionamid mit elementarem Chlor gleichfalls ein Gemisch der beiden vorgenannten 3-Isothiazolinone.

Bei vielen Anwendungen ist es nötig, die 3-Isothiazolinone in Lösungen zu formulieren. Als Lösungsmittel dient vorzugsweise Wasser, aber es werden auch polare organische Lösungsmittel, wie Alkohole, dafür eingesetzt. Einige solcher 3-Isothiazolinonlösungen können schon während der Lagerung, also vor ihrer bestimmungsgemäßen Verwendung, eine chemische Zersetzung der 3-Isothiazolinone zeigen, was eine Verminderung der bioziden Wirksamkeit zur Folge hat.

EP-A-606986 offenbart die Verwendung von Eisensalzen zur Stabilisierung von wässrigen 3-Isothiazolinonlösungen. Säuren werden and benutzt, um Ausfüllungen zu vermeiden.

Gemäß der US-A-3870795 kann diesem Nachteil dadurch begegnet werden, daß man zur chemischen Stabilisierung der 3-Isothiazolinone in Formulierungen Metallnitrate oder Metallnitrite zugibt. Es zeigte sich jedoch, daß diese Art der Stabilisierung bei einigen besonders zersetzungsanfälligen 3-Isothiazolinonen nicht ausreicht. Es können nämlich Ausfällungen und Wirkstoffverluste auftreten und die Verwendung der Lösungen stark beeinträchtigen. Dies kann vermindert werden, indem man solche nitratstabilisierten 3-Isothiazolinonlösungen einer zusätzlichen Stabilisierung unterwirft. Gemäß der EP-B-95907 geschieht das bei einem Gemisch aus 5-Chlor-2-methyl-3-isothiazolinon und 2-Methyl-3-isothiazolinon durch eine Wärmebehandlung während 4 h bei 95°C.

Es hat sich aber herausgestellt, daß auch diese Wärmebehandlung zu Nachteilen führt.

Einerseits bewirkt die relativ hohe Temperatur bei der Wärmebehandlung der 3-Isothiazolinonlösung, daß bestimmte Verunreinigungen, die bei der Isothiazolinonherstellung als Nebenprodukte entstanden sind und noch in der Lösung vorliegen, durch das als Stabilisator zugesetzte Nitrat zu Nitrosaminen nitrosiert werden. Letztere sind aber kanzerogene Stoffe, die auf jeden Fall vermieden werden sollen. Jene Nebenprodukte treten vor allem bei der Herstellung des Isothiazolinons aus dem entsprechenden Dithiodiamid auf. Andererseits führt die relativ hohe Temperatur bei der Wärmebehandlung auch dazu, daß ein Teil des wertvollen 3-Isothiazolinons zersetzt wird und somit als Wirkstoff in der Lösung verlorengeht. Dies ist zusätzlich noch mit der Entstehung von zum Teil noch unbekannten Nebenprodukten verbunden, welche die Qualität der 3-Isothiazolinonlösung verschlechtern.

Der Erfindung liegt somit die Aufgabe zugrunde, ein Verfahren zur Herstellung von qualitativ verbesserten bioziden oder biostatischen Zusammensetzungen, die in Wasser und/oder in einem polaren organischen Lösungsmittel vorliegen, mit einem Gehalt an mindestens einem 3-Isothiazolinon zur Verfügung zu stellen. Die dabei angewandte Stabilierungsmethode soll zu keinen oder nur geringen Wirkstoffverlusten führen, und die erhaltenen Lösungen sollen einen verringerten Gehalt an störenden Nebenprodukten aufweisen, deren Anwesenheit sich auch in Form von Niederschlägen und Färbungen der Zusammensetzungen äußern kann.

Diese Aufgabe löst die Erfindung durch ein Verfahren zur Herstellung von qualitativ verbesserten bioziden oder biostatischen Zusammensetzungen, die in Wasser und/oder in einem polaren organischen Lösungsmittel vorliegen, mit einem Gehalt an mindestens einem 3-Isothiazolinon der allgemeinen Formel 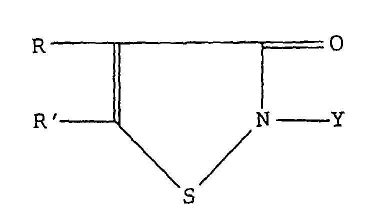 in der
Y ein Wasserstoffatom oder einen unsubstituierten Alkylrest mit 1 bis 18 Kohlenstoffatomen, einen unsubstituierten oder halogensubstituierten Alkenyl- oder Alkinylrest mit 2 bis 8 Kohlenstoffatomen, einen Cycloalkylrest mit 5 bis 8 Kohlenstoffatomen, einen Aralkylrest mit 7 bis 11 Kohlenstoffatomen oder einen Arylrest mit 6 bis 10 Kohlenstoffatomen sowie
R und R' jeweils ein Wasserstoff- oder Halogenatom oder einen C₁- bis C₄-Alkylrest
bedeuten, wobei das 3-Isothiazolinon in an sich bekannter Weise hergestellt und dann durch Zusatz eines Nitrats stabilisiert wird,
dadurch gekennzeichnet,
daß das bei der Herstellung des 3-Isothiazolinons erhaltene Gemisch nach dem Zusatz des Nitrats einer Nachstabilisierung unterzogen wird, indem es bei einem pH-Wert von 0,2 bis 1,5 und einer Temperatur von höchstens 40°C während eines Zeitraums von mindestens 48 h gelagert sowie anschließend wieder auf einen höheren pH-Wert gebracht wird.

In der erfindungsgemäßen Zusammensetzung kann als polares organisches Lösungsmittel beispielsweise ein Alkohol, wie ein Glykol, z.B. Diethylenglykol, Dipropylenglykol und 1,2-Propylenglykol, vorliegen.

Die Konzentration an 3-Isothiazolinon in der erfindungsgemäßen Zusammensetzung kann in einem breiten Bereich liegen, beispielsweise in einem Bereich von 1 bis 30 Gewichtsprozent, vorzugsweise von 10 bis 20 Gewichtsprozent.

Vorzugsweise wird das bei der Herstellung des 3-Isothiazolinons erhaltene Gemisch bei einem pH-Wert von 0,5 bis 1,5 , insbesondere von 0,8 bis 1,2 , und bei einer Temperatur von 0 bis 40 °C, insbesondere von 10 bis 30 °C, während eines Zeitraums von 3 Tagen bis 3 Wochen, insbesondere von 1 bis 2 Wochen, gelagert.

Besonders vorteilhaft ist das erfindungsgemäße Verfahren bei der Anwendung auf ein Gemisch, das als 3-Isothiazolinon 5-Chlor-2-methyl-3-isothiazolinon und/oder 2-Methyl-3-isothiazolinon enthält.

Das erfindungsgemäße Verfahren kann aber ebenso bei Zusammensetzungen anderer 3-Isothiazolinone erfolgreich benutzt werden.

Die Anwendung des erfindungsgemäßen Verfahrens ist vom Weg der Herstellung des 3-Isothiazolinons unabhängig. Dieser Wirkstoff kann also beispielsweise durch Cyclisieren eines Dithioamids oder eines Mercaptoamids oder auf einem anderen bekannten Weg erhalten worden sein.

Durch das erfindungsgemäße Verfahren werden 3-Isothiazolinonlösungen stabilisiert, ohne die bisher übliche Wärmebehandlung bei relativ hohen Temperaturen durchführen zu müssen. Das erfindungsgemäße Verfahren hat damit den Vorteil, daß es mit keinen oder nur geringen Wirkstoffverlusten verbunden ist.

Durch die Vermeidung der Zersetzung des 3-Isothiazolinons bei der erfindungsgemäßen Nachstabilisierungsbehandlung wird auch die Bildung von entsprechenden Zersetzungsprodukten verhindert. Das bedeutet, daß die erfindungsgemäß hergestellten Zusammensetzungen insgesamt weniger Verunreinigungen enthalten als die entsprechenden bekannten Zusammensetzungen nach ihrer Wärmebehandlung.

Die höhere Reinheit der erfindungsgemäß erhaltenen Zusammensetzungen äußert sich auch in ihrem Aussehen. Die Lösungen sind auch nach längerer Lagerung weitgehend oder vollständig klar, weil sie fast oder vollständig frei von Niederschlägen sind. Im Gegensatz dazu bilden die bekannten wärmebehandelten Lösungen während ihrer Aufbewahrung einen Bodensatz, falls sie nicht mit zusätzlichen Stabilisatoren, z. B. mit Kupfersalzen, versetzt worden sind.

Auch die Farbe der erfindungsgemäß hergestellten Zusammensetzungen spiegelt die verbesserte Reinheit wieder. Die Zusammensetzungen sind fast oder vollständig farblos, während die bekannten wärmebehandelten Zusammensetzungen stark gefärbt sind.

Schließlich führt das erfindungsgemäße Verfahren im Vergleich zur bekannten Wärmebehandlung (bei fast 100 °C) naturgemäß zu einer Energieersparnis, weil eine wesentlich niedrigere Temperatur, zum Beispiel Raumtemperatur, bei der Erfindung ausreicht.

Das erfindungsgemäße Verfahren eignet sich insbesondere zur Stabilisierung von 3-Isothiazolinonen, die aufgrund ihres Herstellungsweges noch herstellungsbedingte Verunreinigungen enthalten.

Nach der erfindungsgemäßen Nachstabilisierungsbehandlung im angegebenen pH- und Temperaturbereich während des angegebenen Zeitraums wird der pH-Wert der 3-Isothiazolinonlösung wieder erhöht. Dieser endgültige pH-Wert hängt davon ab, für welchen Anwendungszweck die Lösung auf den Markt gebracht werden soll.

Das Beispiel erläutert die Erfindung.

Alle nachfolgend angegebenen Wirkstoffgehalte wurden mit Hilfe der Hochdruckflüssigkeitschromatographie (HPLC) gemessen. Die Mengenangaben in Prozent beziehen sich auf das Gewicht. Die Lagerung der Proben erfolgte in dicht verschlossenen Glasgefäßen.

### Herstellungsbeispiel

Als Ausgangsmaterial wurde eine wäßrige Zusammensetzung hergestellt, die als Wirkstoff ein Gemisch aus 5-Chlor-2-methyl-3-isothiazolinon und 2-Methyl-3-isothiazolinon enthielt. Die beiden 3-Isothiazolinone wurden in bekannter Weise gemäß der US-A-3849430 erhalten. Dazu wurde N-Methyl-3-mercaptopropionamid mit elementarem Chlor in Butylacetat als Lösungsmittel unter Bildung von 2-Methyl-3-isothiazolinon (MIT) und 5-Chlor-2-methyl-3-isothiazolinon (CIT) cyclisiert.

Die Hydrochloride dieser Produkte wurden durch Filtration abgetrennt und in Wasser aufgenommen.

Die erhaltene Lösung enthielt dann folgende Mengenanteile:
6,3 % MIT
17,3 % CIT

Diese Lösung wurde mit einer wäßrigen Magnesiumoxidaufschlämmung unter Kühlung bei einer Temperatur von 25 bis 27°C auf einen pH-Wert von 0,9 eingestellt und dann zur Stabilisierung mit Magnesiumnitrat versetzt.

Die erhaltene Lösung hatte einen pH-Wert von 0,9 und war klar sowie fast farblos. Sie enthielt die folgenden Stoffe:
3,98 % MIT
10,91 % CIT
15,80 % Magnesiumnitrat
5,50 % Magnesiumchlorid

Diese Lösung diente als Ausgangsmaterial für das nachfolgende Beispiel und die Vergleichsbeispiele 1 bis 3.

### Beispiel

Ein Teil der am Ende des Herstellungsbeispiels vorliegenden Lösung wurde durch Lagern bei einem pH-Wert von 0,9 und einer Temperatur von 25°C während 2 Wochen bei 25°C nachstabilisiert, anschließend mit Magnesiumoxid auf einen pH-Wert von 2,9 eingestellt und filtriert.

Die Wirkstoffgehalte, die Farbzahl (nach Gardner) sowie das Aussehen der so erhaltenen Lösung unmittelbar nach der 2-wöchigen Stabilisierungsbehandlung sind in der nachfolgenden Tabelle I angegeben.

Die Stabilität dieser Lösung nach einer Aufbewahrung während 14 Tagen bei 54°C und alternativ während 3 Monaten bei 20°C ist aus der Tabelle II ersichtlich.

Außerdem wurde die Lösung mit Wasser auf eine handelsübliche Konzentration von 1,5 % Isothiazolinon verdünnt. Die Stabilität dieser verdünnten Lösung ergibt sich aus der Tabelle III.

In der Tabelle I ist der Verlust des Wirkstoffs nur auf das CIT bezogen. Dabei entsprechen 100 % CIT (ohne Wirkstoffverlust) dem im Herstellungsbeispiel angegebenen Mengenanteil von 10,91 % CIT in der Ausgangslösung. Es ist ausreichend, hier nur das CIT (und nicht auch das MIT) zu betrachten, weil das CIT als biozider Wirkstoff wesentlich aktiver ist als das MIT und auch diejenige Komponente im MIT/CIT-Gemisch darstellt, die eine besondere Stabilisierung benötigt.

Aus dem gleichen Grund ist auch in der Tabelle II der Restprozentgehalt des Isothiazolinons in der aufbewahrten Lösung nur auf das CIT bezogen.

### Vergleichsbeispiel 1

Dieses Vergleichsbeispiel veranschaulicht die in der US-A-3870795 beschriebene Stabilisierungsmethode, d. h. die Zugabe eines Nitrats ohne weitere Nachstabilisierung.

Ein Teil der am Ende des Herstellungsbeispiels vorliegenden Lösung wurde keiner weiteren Stabilisierungsbehandlung unterworfen, sondern nach der Herstellung sofort mit Magnesiumoxid auf einen pH-Wert von 2,9 eingestellt sowie filtriert.

Die Wirkstoffgehalte, die Farbzahl, das Aussehen und die Stabilität der Lösung wurden wie im vorstehenden Beispiel bestimmt. Die Ergebnisse sind in entsprechender Weise in den Tabellen I, II und III zusammengefaßt.

### Vergleichsbeispiel 2

Bei diesem Vergleichsbeispiel wurde die in der EP-B-95907 beschriebene Stabilisierungsmethode angewandt, d. h. die Stabilisierung erfolgte durch Zugabe eines Nitrats und durch eine anschließende Wärmebehandlung als Nachstabilisierung.

Ein Teil der am Ende des Herstellungsbeispiels vorliegenden Lösung wurde nach der Herstellung sofort mit Magnesiumoxid auf einen pH-Wert von 2,9 eingestellt, dann zur weiteren Stabilisierung 4 Stunden auf 95° C erwärmt sowie anschließend auf Raumtemperatur abgekühlt und filtriert.

Die Wirkstoffgehalte, die Farbzahl, das Aussehen und die Stabilität der Lösung wurden wie im vorstehenden Beispiel bestimmt. Die Ergebnisse sind in entsprechender Weise in den Tabellen I, II und III zusammengefaßt.

### Vergleichsbeispiel 3

Dieses Vergleichsbeispiel entspricht weitgehend dem Vergleichsbeispiel 2 mit dem Unterschied, daß die Stabilisierung durch Wärmebehandlung bei einem niedrigeren pH-Wert durchgeführt wurde.

Ein Teil der am Ende des Herstellungsbeispiels vorliegenden Lösung wurde nach der Herstellung sofort zur Stabilisierung 4 Stunden auf 95°C erwärmt sowie anschließend mit Magnesiumoxid auf eine pH-Wert von 2,9 eingestellt und filtriert.

Die Wirkstoffgehalte, die Farbzahl, das Aussehen und die Stabilität der Lösung wurden wie im vorstehenden Beispiel bestimmt. Die Ergebnisse sind in entsprechender Weise in den Tabellen I, II und III zusammengefaßt.

Aus den Vergleichbeispielen 1 bis 3 wird deutlich, daß die Stabilisierung mit Magnesiumnitrat allein zu einem wenig verfärbten, aber nicht sehr stabilen Produkt führt. Die Wärmebehandlung bei 95°C verbessert zwar die Stabilität, aber eine deutliche Verfärbung sowie Wirkstoffverluste sind die Folge. Im Gegensatz dazu führt das erfindungsgemäße Beispiel weder zu nennenswerten Wirkstoffverlusten noch zu Verfärbungen, und das Endprodukt weist auch noch eine verbesserte Stabilität auf.

**Tabelle I**

| Vergleich der Lösungen sofort nach ihrer Fertigstellung | | | | | |
|---|---|---|---|---|---|
| | MIT % | CIT % | CIT-Verlust während der Nachstabilisierung % | Farbzahl (Gardner) | Aussehen |
| Beispiel | 3,94 | 10,85 | 0,6 | 1-2 | klar, fast farblos |
| Vergl.-beisp.1 | 3,98 | 10,91 | 0,0 | 1-2 | klar, fast farblos |
| Vergl.-beisp.2 | 3,87 | 10,15 | 6,9 | 5 | klar, gelbbernsteinfarben |
| Vergl.-beisp.3 | 3,73 | 10,22 | 6,3 | 6 | klar, gelbbernsteinfarben |

**Tabelle II**

| Stabilität der unverdünnten Isothiazolinonlösungen | | | |
|---|---|---|---|
| | Rest an CIT nach Aufbewahrung 14 Tage/54°C % | Aussehen der Lösung nach Aufbewahrung 3 Monate/20°C | Farbzahl (Gardner) nach Aufbewahrung 3 Monate/20°C |
| Beispiel | 98,5 | + | 1-2 |
| Vergl.-beisp.1 | 85,3 | - | 2 |
| Vergl.-beisp.2 | 98,8 | o | 5 |
| Vergl.-beisp.3 | 98,2 | o | 6 |
| + = klar, ohne Bodensatz | | | |
| o = leicht trüb mit Bodensatz | | | |
| - = trüb mit Bodensatz | | | |

**Tabelle III**

| Stabilität der 1,5-%igen Isothiazolinonlösungen | | | |
|---|---|---|---|
| | Aussehen der Lösung nach Aufbewahrung | | Farbe |
| | 3 Tage/20°C | 14 Tage/20°C | |
| Beispiel | + | + | farblos |
| Vergl.-beisp.1 | o | - | farblos |
| Vergl.-beisp.2 | o | - | gelb |
| Vergl.-beisp.3 | o | - | gelb |
| + = klar, ohne Bodensatz | | | |
| o = leicht trüb mit Bodensatz | | | |
| - = trüb mit Bodensatz | | | |

## Patentansprüche

1. Verfahren zur Herstellung von qualitativ verbesserten bioziden oder biostatischen Zusammensetzungen, die in Wasser und/oder einem polaren organischen Lösungsmittel vorliegen, mit einem Gehalt an mindestens einem 3-Isothiazolinon der allgemeinen Formel 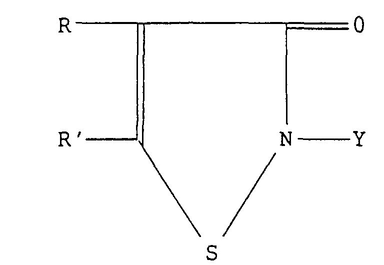 in der
Y ein Wasserstoffatom oder einen unsubstituierten Alkylrest mit 1 bis 18 Kohlenstoffatomen, einen unsubstituierten oder halogensubstituierten Alkenyl- oder Alkinylrest mit 2 bis 8 Kohlenstoffatomen, einen Cycloalkylrest mit 5 bis 8 Kohlenstoffatomen, einen Aralkylrest mit 7 bis 11 Kohlenstoffatomen oder einen Arylrest mit 6 bis 10 Kohlenstoffatomen sowie
R und R' jeweils ein Wasserstoff- oder Halogenatom oder einen C₁- bis C₄-Alkylrest
bedeuten, wobei das 3-Isothiazolinon in an sich bekannter Weise hergestellt und dann durch Zusatz eines Nitrats stabilisiert wird,
dadurch gekennzeichnet,
daß das bei der Herstellung des 3-Isothiazolinons erhaltene Gemisch nach dem Zusatz des Nitrats einer Nachstabilisierung unterzogen wird, indem es bei einem pH-Wert von 0,2 bis 1,5 und einer Temperatur von höchstens 40°C während eines Zeitraums von mindestens 48 h gelagert sowie anschließend wieder auf einen höheren pH-Wert gebracht wird, um das Gemisch seinem Anwendungszweck anzupassen.

2. Verfahren nach Anspruch 1, dadurch gekennzeichnet, daß die Nachstabilisierung bei einem pH-Wert von 0,8 bis 1,2 und einer Temperatur von 10 bis 30°C während eines Zeitraums von 1 bis 2 Wochen erfolgt.

3. Verfahren nach Anspruch 1 oder 2, dadurch gekennzeichnet, daß als 3-Isothiazolinon 5-Chlor-2-methyl-3-isothiazolinon und/oder 2-Methyl-3-isothiazolinon eingesetzt wird.

## Claims

1. Process for the manufacture of qualitatively improved biocidal or biostatic compositions which are present in water and/or a polar organic solvent, with a content of at least one 3-isothiazolinone of the general formula 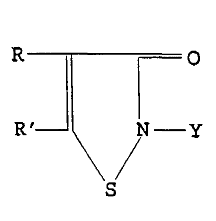 in which Y represents a hydrogen atom or an unsubstituted alkyl group with 1 to 18 carbon atoms, an unsubstituted or halo substituted alkenyl or alkinyl group with 2 to 8 carbon atoms, a cycloalkyl group with 5 to 8 carbon atoms, an aralkyl group with 7 to 11 carbon atoms or an aryl group with 6 to 10 carbon atoms, as well as
R and R' representing in each case a hydrogen or halogen atom or a C₁-C₄ alkyl group,
wherein the 3-isothiazolinone is manufactured in a fashion known per se and then stabilised by the addition of a nitrate, characterised in that
the mixture obtained in the manufacture of the 3-isothiazolinone following the addition of the nitrate is subjected to a post stabilisation in that it is stored at a pH value of 0.2 to 1.5 and a temperature of at most 40°C for a period of time of at least 48 h as well as then being brought again to a higher pH value in order to match the mixture to its desired application.

2. Process according to Claim 1 characterised in that the post stabilisation takes place at a pH value of 0.8 to 1.2 and a temperature of 10 to 30°C during a period of time of 1 to 2 weeks.

3. Process according to Claim 1 or 2 characterised in that used as the 3-isothiazolinone is 5-chloro-2-methyl-3-isothiazolinone and/or 2-methyl-3-isothiazolinone.

## Revendications

1. Procédé pour la préparation de compositions biocides ou biostatiques qualitativement améliorées, qui se présentent dans l'eau et/ou dans un solvant organique polaire, contenant au moins une 3-isothiazolinone de formule générale 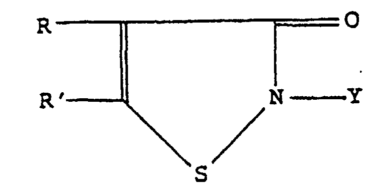 dans laquelle
Y représente un atome d'hydrogène ou un groupe alkyle non substitué ayant de 1 à 18 atomes de carbone, un groupe alcényle ou alcynyle non substitué ou substitué par des halogènes ayant de 2 à 8 atomes de carbone, un groupe cycloalkyle ayant de 5 à 8 atomes de carbone, un groupe aralkyle ayant de 7 à 11 atomes de carbone ou un groupe aryle ayant de 6 à 10 atomes de carbone et
R et R' représentent respectivement un atome d'hydrogène ou d'halogène ou un groupe alkyle en C₁ à C₄,
la 3-isothiazolinone étant préparée de manière connue en soi et ensuite stabilisée par addition d'un nitrate,
caractérisé en ce que,
le mélange obtenu dans la préparation de la 3-isothiazolinone est soumis à une post-stabilisation après l'addition du nitrate, en le stockant à une valeur de pH variant de 0,2 à 1,5 et à une température de 40°C au maximum pendant une durée d'au moins 48 h et en l'amenant ensuite à nouveau à une valeur de pH plus élevée, pour adapter le mélange à son application.

2. Procédé selon la revendication 1, caractérisé en ce que, la post-stabilisation est réalisée à une valeur de pH variant de 0,8 à 1,2 et à une température variant de 10 à 30°C pendant une durée de 1 à 2 semaines.

3. Procédé selon la revendication 1, caractérisé en ce qu'on utilise comme 3-isothiazolinone la 5-chloro-2-méthyl-3-isothiazolinone et/ou la 2-méthyl-3-isothiazolinone.
